# EUROPEAN PATENT APPLICATION

(11) **EP 4 318 417 A1**
(43) Date of publication of application: **07.02.2024**
(21) Application number: 21932969.5
(22) Date of filing: 24.03.2021
(51) Int. Cl.: G07C 9/37

(54) **INFORMATION PROCESSING SYSTEM, INFORMATION PROCESSING DEVICE, INFORMATION PROCESSING METHOD, AND STORAGE MEDIUM**

(71) Applicant: NEC Corporation, 108-8001 Tokyo (JP)
(72) Inventor: IKEDA, Kazufumi, Tokyo 136-8627 (JP); TAKEUCHI, Mamoru, Tokyo 136-8627 (JP)
(74) Representative: Betten & Resch
(86) International application number: PCT/JP2021/012244
(87) International publication number: WO 2022/201359

(57) **Abstract**

An information processing system (SYS) includes: a plurality of gate apparatuses (1) that are respectively placed at a plurality of lanes (L) through each of which a target person (P) can pass; a thermal camera (3) that is configured to generate a body temperature information (IMG_T) indicating a body temperature of the target person by capturing an image of the target person included in an imaging range (TRG) thereof, the imaging range including at least a part of each of the plurality of lanes; and an information processing apparatus (4) that obtains the body temperature information from the thermal camera.

## Description

### Technical Field

The present disclosure relates to a technical field of an information processing system that includes a thermal camera configured to generate a body temperature information indicating a body temperature of a target person by capturing an image of the target person and an information processing apparatus that obtains the body temperature information from the thermal camera, and an information processing apparatus, an information processing method and a recording medium that obtain the body temperature information from the thermal camera, for example.

### Background Art

A Patent Literature 1 discloses a gate apparatus including, as a sensing device that detects a passing person and the like, a thermal sensor that detects a heat such as a body temperature.

Additionally, there are Patent Literatures 2 to 4 as a background art document relating to the present disclosure.

### Citation List

### Patent Literature

Patent Literature 1: JP2019-071126A
Patent Literature 2: JP2001-257927A
Patent Literature 3: JP2009-043046A
Patent Literature 4: JP2020-205117A
Patent Literature 5: JP2020-201999A

### Summary

### Technical Problem

It is an example object of the present disclosure to provide an information processing system, an information processing apparatus, an information processing method and a recording medium that aims to an improvement of a technique disclosed in the background art document.

### Solution to Problem

One example aspect of an information processing system includes: a plurality of gate apparatuses that are respectively placed at a plurality of lanes through each of which a target person can pass; a thermal camera that is configured to generate a body temperature information indicating a body temperature of the target person by capturing an image of the target person included in an imaging range thereof, the imaging range including at least a part of each of the plurality of lanes; and an information processing apparatus that obtains the body temperature information from the thermal camera.

One example aspect of an information processing apparatus includes: an obtaining unit that obtains a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and determining unit that determines, as a passing lane, a lane of the plurality of lanes through which the target person indicated by the body temperature image passes.

One example aspect of an information processing method includes: obtaining a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and determining, as a passing lane, a lane of the plurality of lanes through which the target person indicated by the body temperature image passes.

One example aspect of a recording medium is a recording medium on which a computer program that allows a computer to execute an information processing method is recorded, the information processing method includes: obtaining a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and determining, as a passing lane, a lane of the plurality of lanes through which the target person indicated by the body temperature image passes.

### Brief Description of Drawings

[FIG. 1] FIG. 1 is a block diagram that illustrates an entire configuration of an information processing system in a present example embodiment.
[FIG. 2] FIG. 2 is a planar view that illustrates positions of a plurality of optical cameras, a plurality of gate apparatuses and a thermal camera.
[FIG. 3] FIG. 3 is a block diagram that illustrates a configuration of an information processing apparatus in the present example embodiment.
[FIG. 4] FIG. 4 illustrates one example of a data structure of an entry history DB.
[FIG. 5] FIG. 5 is a flow chart that illustrates an entire flow of an entry management operation that is performed by the information processing apparatus.
[FIG. 6] FIG. 6 conceptually illustrates a thermal image.
[FIG. 7] FIG. 7 conceptually illustrates the thermal image that is divided into a plurality of image areas.
[FIG. 8] FIG. 8 conceptually illustrates the thermal image that is divided into the plurality of image areas.
[FIG. 9] FIG. 9 is a planar view that illustrates a target person passing through a lane together with the optical camera and the thermal camera.
[FIG. 10] FIG. 10 conceptually illustrates the thermal image.
[FIG. 11] FIG. 11 is a planar view that illustrates a plurality of lanes that are physically separated by partition members.
[FIG. 12] FIG. 12 conceptually illustrates the thermal image.
[FIG. 13] FIG. 13 illustrates a position of the thermal camera in a first modified example.
[FIG. 14] FIG. 14 conceptually illustrates a movement of the target person P in a second modified example.
[FIG. 15] FIG. 15 conceptually illustrates the movement of the target person Pin the second modified example.

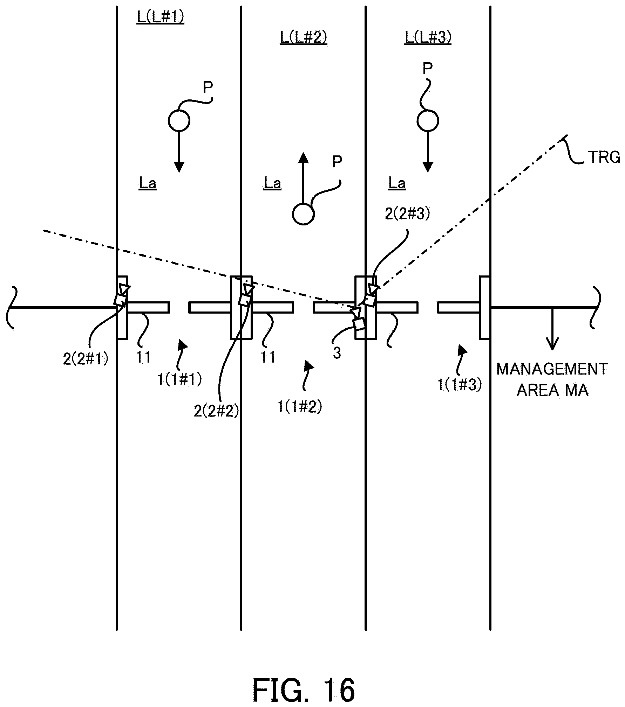

[FIG. 16] FIG. 16 conceptually illustrates the movement of the target person P in the second modified example.
[FIG. 17] FIG. 17 illustrates a position of the optical camera in a third modified example.
[FIG. 18] FIG. 18 is a block diagram that illustrates a configuration of an information processing apparatus in a fourth modified example.
[FIG. 19] FIG. 19 is a block diagram that illustrates a configuration of an information processing system in the fourth modified example.

### Description of Example Embodiments

Next, an example embodiment of an information processing system, an information processing apparatus, an information processing method and a recording medium will be described. In the below described description, the example embodiment of the information processing system, the information processing apparatus, the information processing method and the recording medium will be described by using an information processing system SYS to which the example embodiment of the information processing system, the information processing apparatus, the information processing method and the recording medium is applied.

### (1) Configuration of Information Processing System SYS

Firstly, a configuration of the information processing system SYS in the present example embodiment will be described.

### (1-1) Entire Configuration of Information Processing System SYS

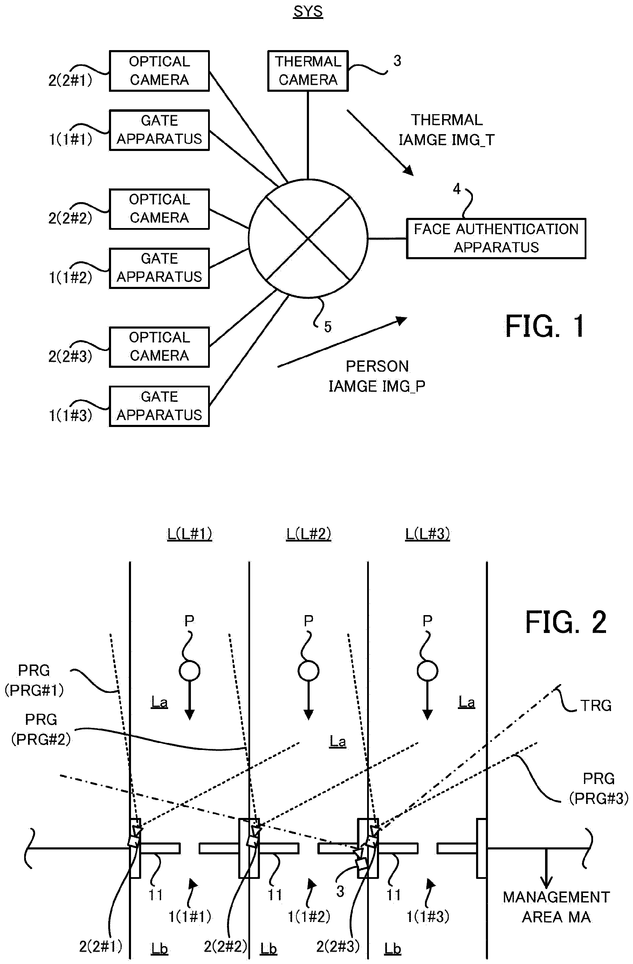

Firstly, with reference to FIG. 1, an entire configuration of the information processing system SYS in the present example embodiment will be described. FIG. 1 is a block diagram that illustrates the entire configuration of the information processing system SYS in the present example embodiment.

As illustrated in FIG. 1, it includes a plurality of gate apparatuses 1, a plurality of optical cameras 2, a thermal camera 3 and an information processing apparatus 4 that is one specific example of an "information processing apparatus". The plurality of gate apparatuses 1 and the information processing apparatus 4 may be configured to communicate with each other through a communication network 5. The plurality of optical cameras 2 and the information processing apparatus 4 may be configured to communicate with each other through the communication network 5. The thermal camera 3 and the information processing apparatus 4 may be configured to communicate with each other through the communication network 5. The communication network 4 may include a wired communication network. The communication network 5 may include a wireless communication network.

The gate apparatus 1 is an apparatus that is configured to control a passing of a target person P. A state of the gate apparatus 1 is switchable between a close state in which the target person P cannot pass through the gate apparatus 1 and an open state in which the target person P can pass through the gate apparatus 1. For example, when the gate apparatus 1 includes a gate bar 11 (see FIG. 2 described later), the state of the gate apparatus 2 may be switchable between the close state in which the gate bar 11 is closed and the open state in which the gate bar 11 is opened.

The plurality of gate apparatuses 1 may be placed at an entrance of a management area MA (see FIG. 2 described later) which the target person P that satisfies a predetermined entry condition is permitted to enter and the target person P that does not satisfy the predetermined entry condition is not permitted (namely, is prohibited) to enter. In this case, when the target person P satisfies the predetermined entry condition, the state of one gate apparatus 1 through which this target person P will pass is switched to the open state. As a result, the target person P that satisfies the predetermined entry condition can enter the management area MA by passing through the gate apparatus 1 that is in the open state. On the other hand, when the target person P does not satisfy the predetermined entry condition, the state of one gate apparatus 1 through which this target person P will pass is switched to the closed state. As a result, the target person P that does not satisfy the predetermined entry condition cannot pass through the gate apparatus 1 that is in the closed state and thus cannot enter the management area MA. However, the plurality of gate apparatuses 1 may be placed at a position that is different from the entrance of the management area MA. In the below described description, an example in which the plurality of gate apparatuses 1 may be placed at the entrance of the management area MA will be described for convenience of description.

The optical camera 2 is an imaging apparatus that is configured to optically capture an image of the target person P that is located in an imaging range PRG of the optical camera 2. The optical camera 2 generates a person image IMG_P that indicates the target person P the image of which is captured by the optical camera 2 by capturing the image of the target person P. The person image IMG_P that indicates the target person P may be typically an image in which the target person P is included. Note that the "person image IMG P in which the target person P is included" may include an image that is generated by means of the optical camera 2 capturing the image of the target person P that does not have an intention of wanting the optical camera 2 to capture the image of the target person P. The "person image IMG_P in which the target person P is included" may include an image that is generated by means of the optical camera 2 capturing the image of the target person P that has the intention of wanting the optical camera 2 to capture the image of the target person P. The optical camera 2 transmits the generated person image IMG_P to the information processing apparatus 4 through the communication network 5.

The gate apparatuses 1 and the plurality of optical cameras 2 are placed so as to have one to one relationship between each other. Thus, the number of the optical cameras 2 is equal to the number of the gate apparatuses 1. Incidentally, in the below described description, an example in which the information processing system SYS includes three gate apparatuses 1 (specifically, the gate apparatus 1#1, the gate apparatus 1#2 and the gate apparatus 1#3) and three optical cameras 2 (specifically, the optical camera 2#1, the optical camera 2#2 and the optical camera 2#3) will be described for convenience of description.

Specifically, as illustrated in FIG. 2 that is a planar view illustrating positions of the gate apparatuses 1#1 to 1#3 and the optical cameras 2#1 to 2#3, each gate apparatus 1 is placed at one lane L through which the target person P can pass in order to pass through each gate apparatus 1. Furthermore, each optical camera 2 is placed so that the imaging range PRG of each optical camera 2 includes at least a part of one lane L through which the target person P can pass in order to pass through one gate apparatus 1 that corresponds to each optical camera 2. Especially, each optical camera 2 is placed so that the imaging range PRG of each optical camera 2 includes at least a part of an area of one lane L, which corresponds to each optical camera 2, through which the target person P can pass before the target person P passes through one gate apparatus 1 that corresponds to each optical camera 2. Namely, each optical camera 2 is placed so as to capture the image of the target person P that moves toward the gate apparatus 1 corresponding to each optical camera 2. In this case, each optical camera 2 is typically placed at or near the gate apparatus 1 that corresponds to each optical camera 2. Moreover, each optical camera 2 is placed so that the imaging range PRG includes at least a part of a lane part La of the lane L, which corresponds to each optical camera 2, through which the target person P passes before passing through the gate apparatus 1 (namely, a lane part that is located toward one side from the gate apparatus 1). On the other hand, each optical camera 2 may not be placed so that the imaging range PRG includes at least a part of a lane part Lb of the lane L, which corresponds to each optical camera 2, through which the target person P passes after passing through the gate apparatus 1 (namely, a lane part that is located toward other side from the gate apparatus 1). Thus, each optical camera 2 captures the image of the target person P that passes through one lane L corresponding to each optical camera 2 before the target person P passes through one gate apparatus 1 corresponding to each optical camera 2. Specifically, the optical camera 2#1 is placed so that the imaging range PRG#1 of the optical camera 2#1 includes at least a part of the lane L#1 through which the target person P, which passes through the gate apparatus 1#1 corresponding to the optical camera 2#1, can pass. Thus, the optical camera 2#1 captures the image of the target person P that passes through the lane L#1 before the target person P passes through the gate apparatus 1#1. Moreover, the optical camera 2#2 is placed so that the imaging range PRG#2 of the optical camera 2#2 includes at least a part of the lane L#2 through which the target person P, which passes through the gate apparatus 1#2 corresponding to the optical camera 2#2, can pass. Thus, the optical camera 2#2 captures the image of the target person P that passes through the lane L#2 before the target person P passes through the gate apparatus 1#2. Moreover, the optical camera 2#3 is placed so that the imaging range PRG#3 of the optical camera 2#3 includes at least a part of the lane L#3 through which the target person P, which passes through the gate apparatus 1#3 corresponding to the optical camera 2#3, can pass. Thus, the optical camera 2#3 captures the image of the target person P that passes through the lane L#3 before the target person P passes through the gate apparatus 1#3.

The lanes L#1 to L#3 (namely, the plurality of lanes) may not be physically separated (in other words, isolated). The lanes L#1 to L#3 may not be physically divided. However, the lanes L#1 to L#3 may be physically separated. For example, the lanes L#1 to L#3 may be physically separated by a structural object (for example, a fence) for physically separating the lanes L#1 to L#3.

Again in FIG. 1, the thermal camera 3 is an imaging apparatus that is configured to capture an image of the target person P that is located in an imaging range TRG of the thermal camera 3. The thermal camera 3 generates a body temperature information that indicates a body temperature of the target person P the image of which is captured by the thermal camera 3 by capturing the image of the target person P. The body temperature information may include an image information (namely, a body temperature image, and it is referred to as a "thermal image IMG_T" hereinafter) that indicates the body temperature of the target person P. The thermal image IMG T may be an image that indicates a distribution of the body temperature of the target person by a color or a contrast. The body temperature information may include a numerical information that quantitatively indicates the body temperature of the target person. The body temperature information may include any data that directly or indirectly indicates the body temperature of the target person. Incidentally, in the below describe description, an example in which the thermal camera 3 generates the thermal image IMG T will be described for convenience of description. Typically, the thermal image IMG_T indicating the body temperature of the target person P may be an image in which the target person P is substantially included by the distribution of the body temperature of the target person P. Note that the "thermal image IMG T in which the target person P is included" may include an image that is generated by means of the thermal camera 3 capturing the image of the target person P that does not have an intention of wanting the thermal camera 3 to capture the image of the target person P. The "thermal image IMG T in which the target person P is included" may include an image that is generated by means of the thermal camera 3 capturing the image of the target person P that has the intention of wanting the thermal camera 3 to capture the image of the target person P. The thermal camera 3 transmits the generated thermal image IMG_T (namely, the generated body temperature information) to the information processing apparatus 4 through the communication network 5.

As illustrated in FIG. 2 that illustrates a position of the thermal camera 3, the thermal camera 3 is placed so that the imaging range TRG of the thermal camera 3 includes at least a part of each of the plurality of lanes L. Namely, the thermal camera 3 is placed so that the imaging range TRG of the thermal camera 3 includes at least a part of the lane L#1, at least a part of the lane L#2 and at least a part of the lane L#3. Especially, the thermal camera 3 is placed so that the imaging range TRG of the thermal camera 3 includes at least a part of an area of each lane L through which the target person P can pass before the target person P passes through one gate apparatus 1 that corresponds to each lane L. Namely, the thermal camera 3 is placed so as to capture the image of the target person P that moves toward the gate apparatus 1. In this case, the thermal camera 3 is typically placed at or near either one of the plurality of gate apparatuses 1. Thus, the thermal camera 3 captures the image of the target person P that passes through each lane L corresponding to each gate apparatus 1 before the target person P passes through each gate apparatus 1. Specifically, the thermal camera 3 captures the image of the target person P that passes through the lane L#1 before the target person P passes through the gate apparatus 1#1. Moreover, the thermal camera 3 captures the image of the target person P that passes through the lane L#2 before the target person P passes through the gate apparatus 1#2. Moreover, the thermal camera 3 captures the image of the target person P that passes through the lane L#3 before the target person P passes through the gate apparatus 1#3.

Again in FIG. 1, the information processing apparatus 4 obtains the person image IMG_P from at least one of the plurality of optical cameras 2. Furthermore, the information processing apparatus 4 obtains the thermal image IMG_T (namely, the body temperature information) from the thermal cameras 3. The information processing apparatus 4 performs a desired operation based on the obtained person image IMG P and thermal image IMG_T (namely, the body temperature information).

In the below described description, an example in which the information processing apparatus 4 performs, as one example of the desired operation, an entry management operation for managing an entry of the target person P to the management area MA at which the gate apparatuses 1#1 to 1#3 are placed. The entry management operation may include an operation for authenticating the target person P included in the person image IMG_P (namely, determining whether or not the target person P is same as a registered person) based on the person image IMG_P. In this case, the entry condition that should be satisfied for the target person P to enter the management area MA may include an authentication condition that the authentication of the target person succeeds (namely, the target person P is same as the registered person). Moreover, the entry management operation may include an operation for determining the body temperature of the authenticated target person P based on the thermal image IMG_T and determining whether or not the determined body temperature is normal (typically, is lower than an allowable upper limit value). In this case, the entry condition that should be satisfied for the target person P to enter the management area MA may include a body temperature condition that the body temperature of the target person is normal.

### (1-2) Configuration of Information Processing Apparatus 4

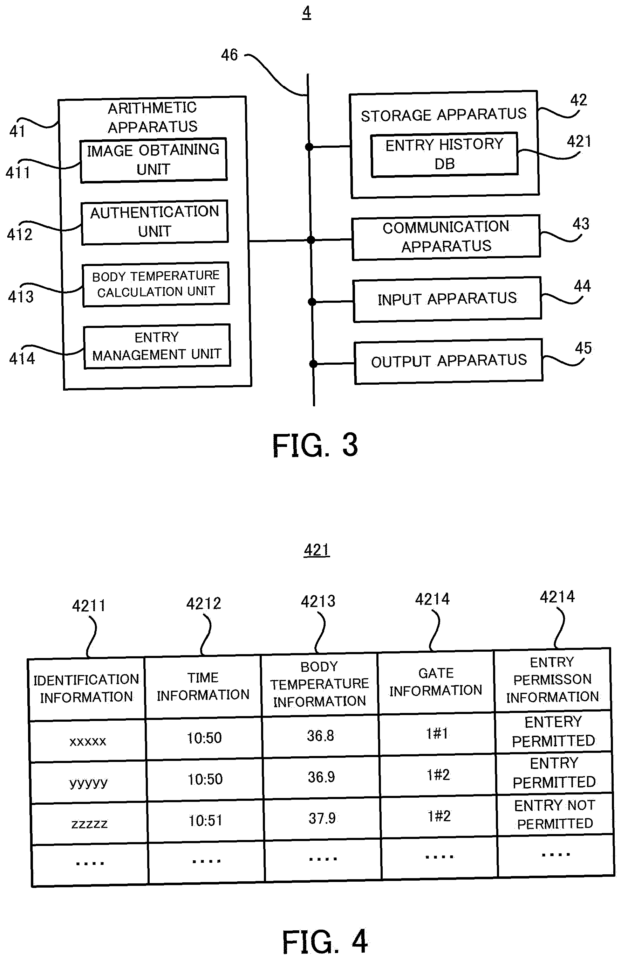

Next, with reference to FIG. 3, a configuration of the information processing apparatus 4 will be described. FIG. 3 is a block diagram that illustrates the configuration of the information processing apparatus 4.

As illustrated in FIG. 3, the information processing apparatus 4 includes an arithmetic apparatus 41, a storage apparatus 42 and a communication apparatus 43. Furthermore, the information processing apparatus 4 may include an input apparatus 44 and an output apparatus 45. However, the information processing apparatus 4 may not include at least one of the input apparatus 44 and the output apparatus 45. The arithmetic apparatus 41, the storage apparatus 42, the communication apparatus 43, the input apparatus 44 and the output apparatus 45 may be interconnected through a data bus 46.

The arithmetic apparatus 41 includes at least one of a CPU (Central Processing Unit), a GPU (Graphic Processing Unit) and a FPGA (Field Programmable Gate Array), for example. The arithmetic apparatus 41 reads a computer program. For example, the arithmetic apparatus 41 may read a computer program that is stored in the storage apparatus 42. For example, the arithmetic apparatus 41 may read a computer program that is stored in a non-transitory computer-readable recording medium by using a non-illustrated recording medium reading apparatus of the information processing apparatus 4. The arithmetic apparatus 41 may obtain (namely, download or read) a computer program from a non-illustrated apparatus that is placed outside the information processing apparatus 4 through the communication apparatus 43 (alternatively, other communication apparatus) The arithmetic apparatus 41 executes the read computer program. As a result, a logical functional block for performing an operation (for example, the above described entry management operation) that should be performed by the information processing apparatus 4 is implemented in the arithmetic apparatus 41. Namely, the arithmetic apparatus 41 is configured to serve as a controller for implementing the logical functional block for performing the operation (in other words, a processing) that should be performed by the information processing apparatus 4.

FIG. 3 illustrates one example of the logical functional block that is implemented in the arithmetic apparatus 41 for performing the entry management operation. As illustrated in FIG. 3, in the arithmetic apparatus 41, an image obtaining unit 411 that is one specific example of "an obtaining unit", an authentication unit 412 that is one specific example of "an authenticating unit", a body temperature calculation unit 413 that is one specific example of "a determining unit" and an entry management unit 414 that is one specific example of "a generating unit" are implemented. Note that a detail of an operation of each of the image obtaining unit 411, the authentication unit 412, the body temperature calculation unit 413 and the entry management unit 414 will be described later in detail, however, a summary thereof will be described briefly here. The image obtaining unit 411 obtains the person image IMG_P from at least one of the plurality of optical cameras 2. Furthermore, the obtaining unit 411 obtains the thermal image IMG_T from the thermal camera 3. The authentication unit 412 authenticates the target person P included in the person image IMG_P (namely, determining whether or not the target person P is same as the registered person) based on the person image IMG_P. The body temperature calculation unit 413 calculates, based on the thermal image IMG_T, the body temperature of the target person P that is authenticated by the authentication unit 412. The entry management unit 414 manages the entry of the target person P to the management area MA. Furthermore, the entry management unit 414 generates an entry history DB (DataBase) 421 for managing an entry history of the target person P to the management area MA.

FIG. 4 illustrates one example of a data structure of the entry history DB 421. As illustrated in FIG. 4, it includes a history record 4210. The history record 4210 may include an identification information 4211 for identifying the target person P, a time information 4212 that indicates a time at which the target person P is authenticated, a body temperature information 4213 that indicates the body temperature of the target person P, a gate information 4214 for identifying the gate apparatus 1 through which the target person P has passed (alternatively, tried to pass), and an entry permission information 4215 that indicates whether or not entry of the target person P has been permitted. In this manner, the identification information 4211 for identifying the target person P is associated with the body temperature information 4213 that indicates the body temperature of the target person P in the history record 4210. Note that an information indicating a name of the target person P is used as the identification information 4211 in an example illustrated in FIG. 4.

Again in FIG. 3, the storage apparatus 42 is configured to store a desired data. For example, the storage apparatus 42 may temporarily store the computer program that is executed by the arithmetic apparatus 41. The storage apparatus 42 may temporarily store a data that is temporarily used by the arithmetic apparatus 41 when the arithmetic apparatus 41 executes the computer program. The storage apparatus 42 may store a data that is stored for a long term by the information processing apparatus 4. Especially in the present example embodiment, the storage apparatus 42 may store the entry history DB 421. Note that the storage apparatus 42 may include at least one of a RAM (Random Access Memory), a ROM (Read Only Memory), a hard disk apparatus, a magneto-optical disc, a SSD (Solid State Drive) and a disk array apparatus. Namely, the storage apparatus 42 may include a non-transitory recording medium.

The communication apparatus 43 is configured to communicate with the plurality of optical cameras 2 and the thermal camera 3 through the communication network 5. In the present example embodiment, the communication apparatus 43 receives (namely, obtains) the person image IMG_P from at least one of the plurality of optical cameras 2 through the communication network 5. Furthermore, the communication apparatus 43 receives (namely, obtains) the thermal image IMG_T from the thermal camera 3 through the communication network 5.

The input apparatus 44 is an apparatus that receives an input of an information from an outside of the information processing apparatus 4 to the information processing apparatus 4. For example, the input apparatus 44 may include an operational apparatus (for example, at least one of a keyboard, a mouse and a touch panel) that is operable by an operator of the information processing apparatus 4. For example, the input apparatus 44 may include a reading apparatus that is configured to read an information recorded as a data in a recording medium that is attachable to the information processing apparatus 4.

The output apparatus 45 is an apparatus that outputs an information to an outside of the information processing apparatus 4. For example, the output apparatus 45 may output the information as an image. Namely, the output apparatus 45 may include a display apparatus (what we call a display) that is configured to display the image representing the information to be outputted. For example, the output apparatus 45 may output the information as a sound. Namely, the output apparatus 45 may include an audio apparatus (what we call a speaker) that is configured to output the sound. For example, the output apparatus 45 may output the information on a paper. Namely, the output apparatus 45 may include a print apparatus (what we call a printer) that is configured to print a desired information on the paper.

### (2) Entry Management Operation performed by Information Processing Apparatus 4

Next, the entry management operation that is performed by the information processing apparatus 4 will be described.

### (2-1) Entire Flow of Entry Management Operation

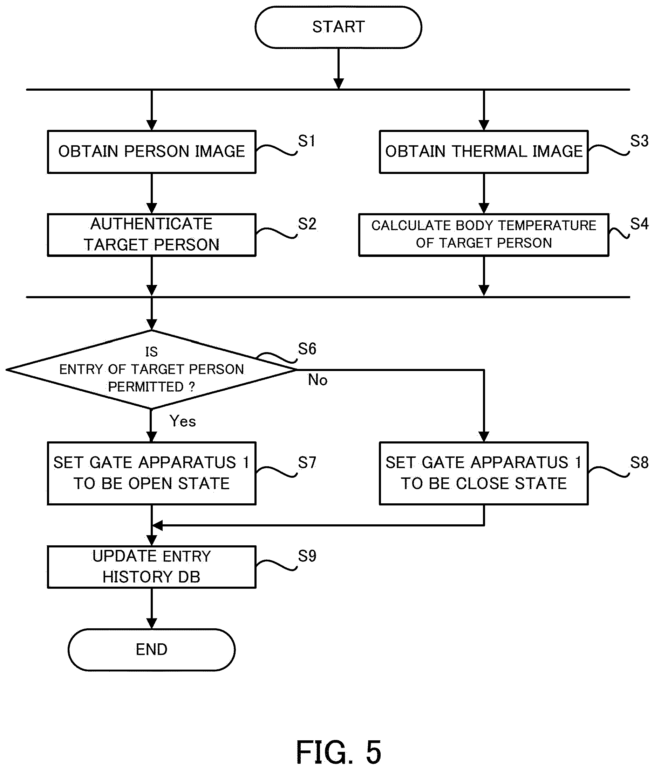

Firstly, with reference to FIG. 5, an entire flow of the entry management operation that is performed by the information processing apparatus 4 will be described. FIG. 5 is a flowchart that illustrates the entire flow of the entry management operation that is performed by the information processing apparatus 4.

As illustrated in FIG. 5, the image obtaining unit 411 obtains the person image IMG_P from at least one of the plurality of optical cameras 2 (a step S1). Then, the authentication unit 412 authenticates the target person P that is included in the person image IMG_P obtained at the step S1 based on the person image IMG_P obtained at the step S1 (a step S2). Specifically, the authentication unit 412 authenticates the target person P that passes through the lane L#1 (namely, the target person P that moves toward the gate apparatus 1#1 in order to pass through the gate apparatus 1#1) based on the person image IMG_P obtained from the optical camera 2#1. Similarly, the authentication unit 412 authenticates the target person P that passes through the lane L#2 (namely, the target person P that moves toward the gate apparatus 1#2 in order to pass through the gate apparatus 1#2) based on the person image IMG_P obtained from the optical camera 2#2. Similarly, the authentication unit 412 authenticates the target person P that passes through the lane L#3 (namely, the target person P that moves toward the gate apparatus 1#3 in order to pass through the gate apparatus 1#3) based on the person image IMG_P obtained from the optical camera 2#3.

The authentication unit 412 may perform a face authentication operation for authenticating the target person P by using a feature point of a face of the target person P included in the person image IMG_P. The authentication unit 412 may perform an iris authentication operation for authenticating the target person P by using a feature point of an iris of the target person P included in the person image IMG P.

In parallel with the operation from the step S1 to the step S2, the image obtaining unit 411 obtains the thermal image IMG_T from the thermal camera 3 (a step S3). Then, the body temperature calculation unit 413 calculates, based on the thermal image IMG T obtained at the step S3, the body temperature of the target person P that is authenticated at the step S2 (a step S4)

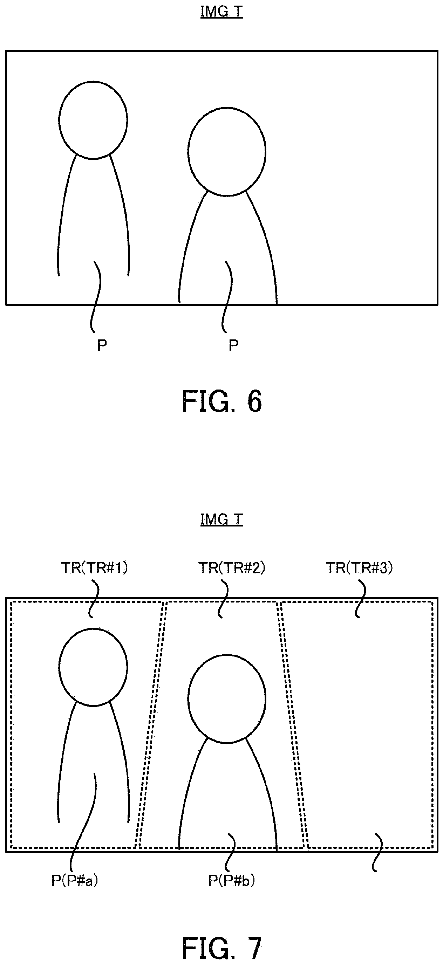

Here, as described above, the imaging range TRG of the thermal camera 3 includes at least a part of each of the plurality of lanes L. Thus, as illustrated in FIG. 6 that conceptually illustrates the thermal image IMG_T, there is a possibility that the target person P that is passing through either one of the lanes #1 to L#3 and the target person P that is passing through another one of the lanes #1 to L#3 are included in the thermal image IMG T at the same time. Namely, there is a possibility that the plurality of target persons P that pass through the plurality of different lanes L, respectively, are included in the thermal image IMG_T. Thus, in the present example embodiment, the body temperature calculation unit 413 determines one lane L through which each target person P included in the thermal image IMG_T is passing through in order to calculate the body temperature of the target person P authenticated at the step S2. Then, the body temperature calculation unit 413 calculates, based on the thermal image IMG_T, the body temperature of each target person P that is passing through the determined one lane L as the body temperature of each target person P that is authenticated based on the person image IMG_P obtained from one optical camera 2 corresponding to the determined one lane L. Specifically, when the person image IMG P is obtained from the optical camera 2#1 at the step S2, the target person P included in the person image IMG_P is passing through the lane L#1. In this case, the body temperature calculation unit 413 determines the target person P that is passing through the lane L#1 in the thermal image IMG_T by determining one lane L through which each target person P included in the thermal image IMG_T is passing, and calculates the body temperature of the determined target person P as the body temperature of the target person P that is passing through the lane L# 1 (namely, the target person P that is included in the person image IMG_P obtained from the optical camera 2#1). The same applies to a case where the person image IMG P is obtained from the optical camera 2#2 or 2#3 at the step S2. As a result, even when the thermal camera 3 captures the image of the plurality of target persons P that pass through the plurality of different lanes L, respectively, the body temperature calculation unit 413 is capable of properly calculating the body temperatures of the plurality of target persons P that pass through the plurality of different lanes L, respectively.

Note that the operation for determining one lane L through which each target person P included in the thermal image IMG_T is passing will be described later in detail with reference to FIG. 7 and so on, and thus a description thereof is omitted here.

Again in FIG. 5, then, the entry management unit 414 determines whether or not to permit the target person P, which is include in the person image IMG_P obtained at the step S1, to enter the management area MA (a step S6). Specifically, when the authentication of the target person P succeeds at the step S2 and the body temperature of the target person calculated at the step S4 is normal (typically, is lower than the allowable upper limit value), the entry management unit 414 may determine to permit the target person P to enter the management area MA. On the other hand, when the authentication of the target person P does not succeed at the step S2 and / or the body temperature of the target person calculated at the step S4 is not normal (typically, is higher than the allowable upper limit value), the entry management unit 414 may determine not to permit the target person P to enter the management area MA.

As a result of the determination at the step S6, when it is determined that the target person P is permitted to enter the management area MA (the step S6: Yes), the entry management unit 414 controls the state of one gate apparatus 1 so that the state of one gate apparatus 1 through which the target person P will pass (namely, one gate apparatus 1 corresponding to one optical camera 2 that generates the person image IMG_P in which the target person P is included) becomes the open state (a step S7). For example, the entry management unit 414 may transmits, to one gate apparatus 1, a control signal for setting the state of one gate apparatus 1 to be the open state through the communication network 5. As a result, the target person P can enter the management area MA by passing through the gate apparatus 1 that is in the open state.

On the other hand, as a result of the determination at the step S6, when it is determined that the target person P is not permitted to enter the management area MA (the step S6: No), the entry management unit 414 controls the state of one gate apparatus 1 so that the state of one gate apparatus 1 through which the target person P will pass (namely, one gate apparatus 1 corresponding to one optical camera 2 that generates the person image IMG_P in which the target person P is included) becomes the close state (a step S8). For example, the entry management unit 414 may transmits, to one gate apparatus 1, a control signal for setting the state of one gate apparatus 1 to be the close state through the communication network 5. As a result, the target person P cannot pass through the gate apparatus 1 that is in the close state and thus cannot enter the management area MA.

Then, the entry management unit 414 updates the entry history DB 421 (a step S9). Specifically, the entry management unit 414 adds, to the entry history DB 421, a new history record 4210 that includes an information relating to an authenticated result of the target person at the step S2 (specifically, the identification information 4211, the time information 4212 and the authentication information 4214 described above), an information relating to the body temperature of the target person P calculated at the step S4 (specifically, the body temperature information 4213) and an information relating to a determined result at the step S6 (specifically, the entry permission information 4215).

### (2-2) Specific Example of Operation For Determining One Lane L Through Which Target Person Included In Thermal Image IMG T Is Passing Through

Next, the operation for determining one lane L through which each target person P included in the thermal image IMG_T is passing will be described.

### (2-2-1) First Specific Example

Firstly, with reference to FIG. 7, a first specific example of the operation for determining one lane L through which each target person P included in the thermal image IMG T is passing will be described. FIG. 7 conceptually illustrates the thermal image IMG_T.

As illustrated in FIG. 7, in the first specific example, the body temperature calculation unit 413 divides the thermal image IMG_T into a plurality of image areas TR (specifically, image areas TR#1 to TR#3) that corresponds to the lanes L#1 to L#3 (namely, the plurality of lanes L). The image area TR#1 is an image area in which the target person P passing through the lane L#1 is expected to be included in the thermal image IMG_T. The image area TR#2 is an image area in which the target person P passing through the lane L#2 is expected to be included in the thermal image IMG_T. The image area TR#3 is an image area in which the target person P passing through the lane L#3 is expected to be included in the thermal image IMG_T.

Then, the body temperature calculation unit 413 determines, as one lane L through which each target person P is passing, one lane L that corresponds to one image area TR of the image areas TR#1 to TR#3 in which each target person P is included. For example, in an example illustrated in FIG. 7, the target person P#a is included in the image area TR#1 and the target person P#b is included in the image area TR#2. In this case, the body temperature calculation unit 413 determines the lane #1 as the lane L through which the target person P#a is passing. As a result, the body temperature calculation unit 413 calculates, based on the thermal image IMG_T, the body temperature of the target person P#a included in the thermal image IMG_T as the body temperature of the target person P that is passing through the lane L#1 (namely, the target person P that is included in the person image IMG_P obtained from the optical camera 2#1). Similarly, the body temperature calculation unit 413 determines the lane #2 as the lane L through which the target person P#b is passing. As a result, the body temperature calculation unit 413 calculates, based on the thermal image IMG_T, the body temperature of the target person P#b included in the thermal image IMG T as the body temperature of the target person P that is passing through the lane L#2 (namely, the target person P that is included in the person image IMG_P obtained from the optical camera 2#2).

The body temperature calculation unit 413 may divide the thermal image IMG T into the image areas TR#1 to TR#3 based on positions of the lanes L#1 to L#3 (namely, the positions of the gate apparatuses 1#1 to 1#3), the position of the thermal camera 3 and an optical characteristic (for example, the imaging range TRG) of the thermal camera 3. Thus, in the first specific example, it is preferable that an information relating to the positions of the lanes L#1 to L#3 (namely, the positions of the gate apparatuses 1#1 to 1#3), the position of the thermal camera 3 and the optical characteristic (for example, the imaging range TRG) of the thermal camera 3 be an information that is known to the body temperature calculation unit 413.

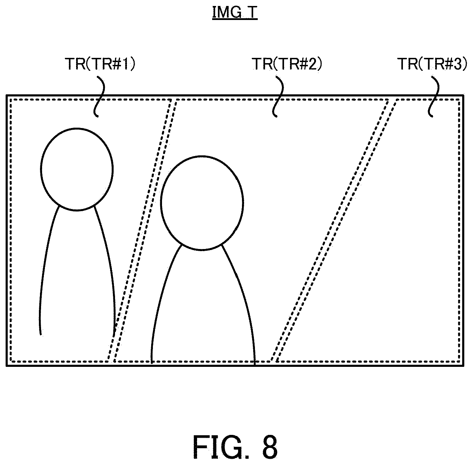

A characteristic of at least one of the image areas TR#1 to TR#3 may be fixed. Note that the characteristic of the image area TR may include at least one of a size of the image area TR and a shape of the image area TR. Alternatively, the body temperature calculation unit 413 may change the characteristic of at least one of the image areas TR#1 to TR#3. For example, when at least one of the positions of the lanes L#1 to L#3 (namely, the positions of the gate apparatuses 1#1 to 1#3), the position of the thermal camera 3 and the optical characteristic (for example, the imaging range TRG) of the thermal camera 3 changes, the body temperature calculation unit 413 may change the characteristic of at least one of the image areas TR#1 to TR#3. As one example, FIG. 7 illustrates one example of the image areas TR#1 to TR#3 when the thermal camera 2 is placed at the lane L#2 that is located between the lane L#1 and the lane L#3, for example. On the other hand, when the thermal camera 3 is placed at the lane L#1, the body temperature calculation unit 413 may divide the thermal image IMG_T into the image areas TR#1 to TR#3 illustrated in FIG. 8 the characteristics of which are different from those of the image areas TR#1 to TR#3 illustrated in FIG. 7.

### (2-2-2) Second Specific Example

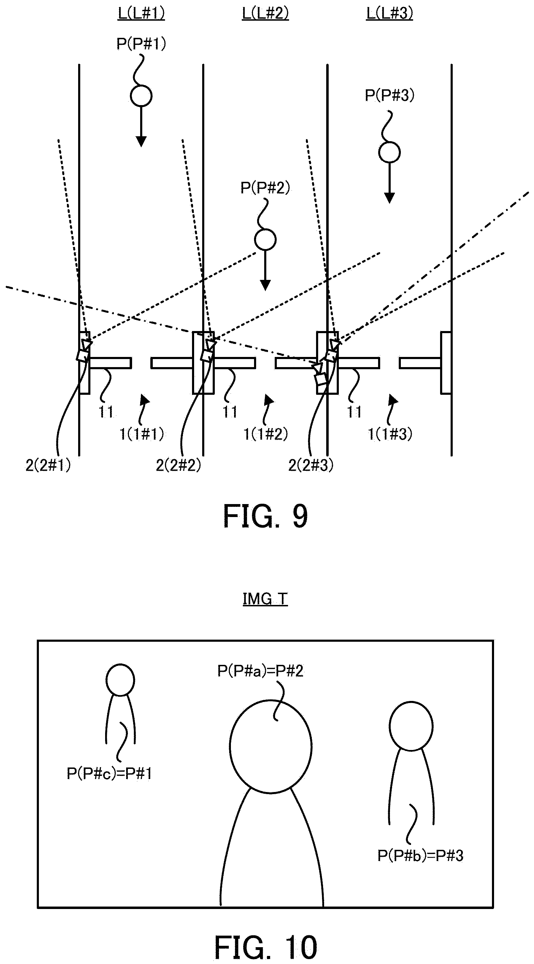

Next, with reference to FIG. 9, a second specific example of the operation for determining one lane L through which each target person P included in the thermal image IMG T is passing will be described. FIG. 9 is a planar view that illustrates the target persons P#1 to P#3 that pass through the lanes L#1 to L#3, respectively, together with the optical cameras 2#1 to 2#3 and the thermal camera 3.

As illustrated in FIG. 9, the optical cameras 2#1 to 2#3 respectively capture the images of the target persons P#1 to P#3 that respectively pass through the lanes L#1 to L#3. In this case, the body temperature calculation unit 413 calculates, in a world coordinate system for representing a position in a space in which the lane L#1 to L#3 are provided (namely, the gate apparatuses 1#1 to 1#3 are placed), a position of the target person P#k included in the person image IMG_P based on the person image IMG_P that is generated by the optical camera 2#k (note that k is a variable number indicating 1, 2 or 3). In order to calculate the position of the target person P#k in the world coordinate system, the body temperature calculation unit 413 calculates, in a person image coordinate system for representing a position in the person image IMG_P, a position of a right eye (alternatively, another part that is different from the right eye) of the target person P#k based on the person image IMG_P, for example. Then, the body temperature calculation unit 413 calculates the position of the target person P#k in the world coordinate system based on the position of the right eye of the target person P#k, the positions of the lanes L#1 to L#3 (namely, the positions of the gate apparatuses 1#1 to 1#3), the position of the optical camera 2#k, a characteristic (for example, the imaging range PRG#k) of the optical camera 2#k and an average distance between eyes (namely, an average value of a distance between both eyes of a human). As a result, the body temperature calculation unit 413 is capable of accurately determining whether or not the target person P#k included in the person image IMG_P is the target person P that is passing through the lane L#k (namely, is the target person P that is moving toward the gate apparatus 1#k placed at the lane L#k). Namely, even when the target person that is different from the target person P#k (specifically, the target person passing through the lane L that is different from the lane L#k) is included in the person image IMG_P that is generated by the optical camera 2#k, the target person P#k that is passing through the lane L#k is extractable from the person image IMG_P. Note that one example of technique for extracting the target person P#k that is passing through the lane L#k is disclosed in the Patent Literature 5, for example.

Then, the body temperature calculation unit 413 calculates a timing at which the target person P#k passes through the lane L#k based on the position of the target person P#k I the world coordinate system. Specifically, the body temperature calculation unit 413 calculates a timing at which the target person P#k reaches the gate apparatus 1#k placed at the lane L#k based on the position of the target person P#k I the world coordinate system. Then, the body temperature calculation unit 413 determines one lane L through which each target person P included in the thermal image IMG T is passing based on the calculated timing and the thermal image IMG T. Specifically, as illustrated in FIG. 10 that illustrates one example of the thermal image IMG_T, each target person P#k is included in the thermal image IMG_T to exist in a position nearer to a front side as a distance between the thermal camera 3 and each target person P#k becomes shorter, because the thermal camera 3 captures the image of the target person P#k that moves toward the gate apparatus 1#k. Typically, a size of each target person P#k included in the thermal image IMG_T becomes larger as the distance between the thermal camera 3 and each target person P#k becomes shorter. Thus, the distance between the thermal camera 3 and each target person P#k becomes shorter as the target person P#k reaches the gate apparatus 1#k earlier, and thus, each target person P#k is included in the thermal image IMG_T to exist in the position nearer to the front side. Therefore, since the position in the thermal image IMG T at which each target person P#k exists has a correlation with the timing at which the target person P#k reaches the gate apparatus 1#k, the body temperature calculation unit 413 is capable of determining one lane L through which each target person P included in the thermal image IMG T is passing based on the thermal image IMG_T and the timing at which the target person P#k reaches the gate apparatus 1#k.

In an example illustrated in FIG. 9, the body temperature calculation unit 413 recognizes that a timing at which the target person P#2 reaches the gate apparatus 1#2 is the earliest, a timing at which the target person P#3 reaches the gate apparatus 1#3 is the second earliest and a timing at which the target person P#1 reaches the gate apparatus 1#1 is the latest, by calculating the timings at which the target persons P#1 to P#3 reach the gate apparatuses 1#1 to 1#3, respectively. In this case, the target person P#a is included in the thermal image IMG_T illustrated in FIG. 10 to exist in the position that is nearest to the front side. Thus, the body temperature calculation unit 413 determines the lane L#2 through which the target person P#2, which reaches the gate apparatus 1 at the earliest timing, is passing as one lane L through which the target person P#a included in the thermal image IMG T is passing. As a result, the body temperature calculation unit 413 calculates, based on the thermal image IMG_T, the body temperature of the target person P#a included in the thermal image IMG T as the body temperature of the target person P#2 that is passing through the lane L#2 (namely, the target person P#2 that is included in the person image IMG_P obtained from the optical camera 2#2). Moreover, the target person P#b is included in the thermal image IMG_T illustrated in FIG. 10 to exist in the position that is second nearest to the front side. Thus, the body temperature calculation unit 413 determines the lane L#3 through which the target person P#3, which reaches the gate apparatus 1 at the second earliest timing, is passing as one lane L through which the target person P#b included in the thermal image IMG_T is passing. As a result, the body temperature calculation unit 413 calculates, based on the thermal image IMG_T, the body temperature of the target person P#b included in the thermal image IMG_T as the body temperature of the target person P#3 that is passing through the lane L#3 (namely, the target person P#3 that is included in the person image IMG_P obtained from the optical camera 2#3). Moreover, the target person P#c is included in the thermal image IMG T illustrated in FIG. 10 to exist in the position that is farthest from the front side. Thus, the body temperature calculation unit 413 determines the lane L#1 through which the target person P#1, which reaches the gate apparatus 1 at the latest timing, is passing as one lane L through which the target person P#c included in the thermal image IMG T is passing. As a result, the body temperature calculation unit 413 calculates, based on the thermal image IMG_T, the body temperature of the target person P#c included in the thermal image IMG_T as the body temperature of the target person P#1 that is passing through the lane L#1 (namely, the target person P#1 that is included in the person image IMG_P obtained from the optical camera 2#1).

### (2-2-3) Third Specific Example

Next, a third specific example of the operation for determining one lane L through which each target person P included in the thermal image IMG T is passing will be described. In the third specific example, the body temperature calculation unit 413 calculates the position of the target person P included in the thermal image IMG T in the world coordinate system based on the thermal image IMG_T. An operation for calculating the position of the target person P included in the thermal image IMG T may be same as an operation for calculating the position of the target person P included in the person image IMG_P described in the second specific example. Namely, the body temperature calculation unit 413 may calculate, in a thermal image coordinate system for representing a position in the thermal image IMG_T, a position of the right eye (alternatively, another part that is different from the right eye) of the target person P#k based on the thermal image IMG_T. Then, the body temperature calculation unit 413 mayt calculate the position of the target person P#k in the world coordinate system based on the position of the right eye of the target person P#k, the positions of the lanes L#1 to L#3 (namely, the positions of the gate apparatuses 1#1 to 1#3), the position of the thermal camera 3, the characteristic (for example, the imaging range TRG) of the thermal camera 3 and the average distance between eyes (namely, the average value of the distance between both eyes of the human).

As a result, the body temperature calculation unit 413 is capable of accurately determining whether or not the target person P#k included in the thermal image IMG_T is the target person P that is passing through the lane L#k (namely, is the target person P that is moving toward the gate apparatus 1#k placed at the lane L#k). Namely, the body temperature calculation unit 413 is capable of accurately determining the lane L through which the target person P included in the thermal image IMG T is passing.

Incidentally, when the position of the target person P in the world coordinate system is calculated, it can be said that the body temperature calculation unit 413 substantially calculates the distance between the thermal camera 3 and the target person P. Namely, it can be said that the body temperature calculation unit 413 substantially calculates a distance between the gate apparatus 1 and the target person P. In this case, the body temperature calculation unit 413 may perform an operation considering the distance between the gate apparatus 1 and the target person P. For example, the body temperature calculation unit 413 may calculate the body temperature of the target person P that is included in the thermal image IMG_T and that is so close to the gate apparatus that the distance between the gate apparatus 1 and the target person P is shorter than a predetermined distance. On the other hand, the body temperature calculation unit 413 may not calculate the body temperature of the target person P that is included in the thermal image IMG_T and that is so far from the gate apparatus until the distance between the gate apparatus 1 and the target person P is longer than the predetermined distance. This is because the target person P takes more time to reach the gate apparatus 1 and a necessity for calculating the body temperature of the target person is relatively low.

Alternatively, as described in the second specific example, each target person P is included in the thermal image IMG_T to exist in the position nearer to the front side as the distance between the gate apparatus 1 and each target person P becomes shorter. Typically, the size of each target person P included in the thermal image IMG T becomes larger as the distance between the gate apparatus 1 and each target person P becomes shorter. Therefore, it can be said that a state (for example, a size) of the target person P included in the thermal image IMG_T includes an information relating to the distance between the gate apparatus 1 and the target person P. Thus, the body temperature calculation unit 413 may perform the operation considering the distance between the gate apparatus 1 and the target person P based on the state (for example, the size) of the target person P included in the thermal image IMG_T without calculating the position of the target person P in the world coordinate system based on the thermal image IMG_T.

### (2-2-4) Fourth Specific Example

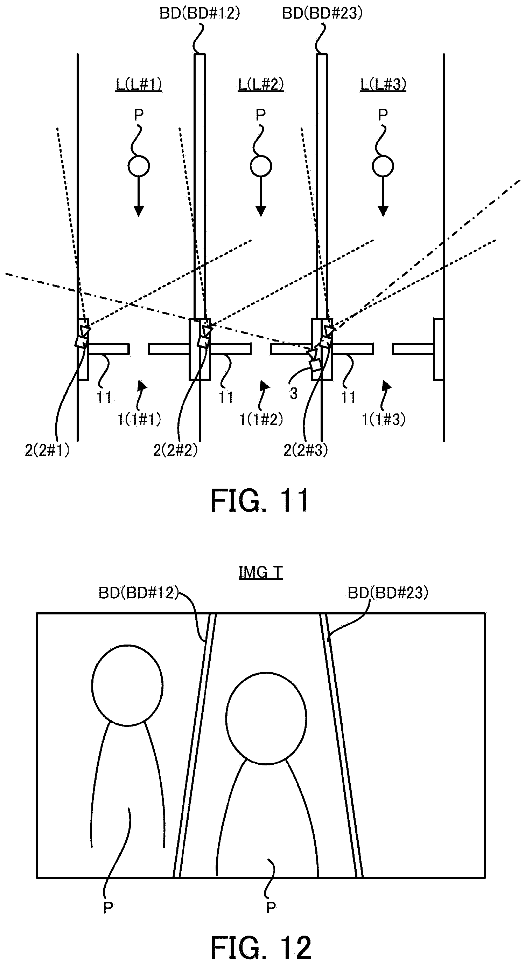

Next, with reference to FIG. 11, a fourth specific example of the operation for determining one lane L through which each target person P included in the thermal image IMG T is passing will be described. FIG. 11 is a planar view that illustrates the lanes L#1 to L#3.

As illustrated in FIG. 11, in the fourth specific example, adjacent two lanes L of the lanes L#1 to L#3 (namely, the plurality of lanes L) are physically separated by a partition member BD. Specifically, adjacent lanes L#1 and L#2 are physically separated by a partition member BD#12 that is placed at a border between the lanes L#1 and L#2. Adjacent lanes L#2 and L#3 are physically separated by a partition member BD#23 that is placed at a border between the lanes L#2 and L#3.

A temperature of the partition member BD is set to be a temperature that is different from a temperature of a space in which the lanes L#1 to L#3 are provided (namely, the gate apparatus 1#1 to 1#3 are placed). For example, the partition member BD may be a member that is less susceptible to the temperature of the space in which the partition member BD is placed. In this case, even when an operation for adjusting the temperature of the partition member BD is not performed, there is a higher possibility that the temperature of the partition member BD is the temperature that is different from the temperature of the space in which the partition member BD is placed. Alternatively, the temperature of the partition member BD may be adjusted by a temperature adjustment apparatus that is configured to adjust the temperature of the partition member BD. The temperature adjustment apparatus may include at least one of an air cooling apparatus and a water cooling apparatus. In this case, the temperature adjustment apparatus may adjust the temperature of the partition member BD so that the temperature of the partition member BD is the temperature that is different from the temperature of the space in which the partition member BD is placed

When the partition member BD is placed, not only the target person P but also the partition member BD are included in the thermal image IMG_T. In this case, as illustrated in FIG. 12 that illustrates the thermal image IMG T in which the partition member BD is included, the partition member BD is included in the thermal image IMG T in an aspect in which the partition member BD is distinguishable. This is because the temperature of the partition member BD is the temperature that is different from the temperature of the space in which the partition member BD is placed as described above.

As a result, the body temperature calculation unit 413 is capable of determining one lane L through which the target person P included in the thermal image IMG T is passing based on a positional relationship between the partition member BD and the target person P in the thermal image IMG_T. Namely, the body temperature calculation unit 413 is capable of determining one lane L through which the target person P included in the thermal image IMG_T is passing based on a of the partition member BD and the position of the target person P in the thermal image IMG T. For example, in an example illustrated in FIG. 12, when the target person P is included at the left of the partition member BD#12 in the thermal image IMG_T, the body temperature calculation unit 413 is capable of determining the lane L#1 as one lane L through which the target person P is passing. For example, when the target person P is included between the partition member BD#12 and the partition member BD#23 in the thermal image IMG_T, the body temperature calculation unit 413 is capable of determining the lane L#2 as one lane L through which the target person P is passing. For example, when the target person P is included at the right of the partition member BD#23 in the thermal image IMG_T, the body temperature calculation unit 413 is capable of determining the lane L#3 as one lane L through which the target person P is passing.

### (3) Technical Effect of Information Processing System SYS

As described above, in the information processing system SYS in the present example embodiment, at least a part of each of the plurality of lanes L are included in the imaging range TRG of the thermal camera 3. Thus, the thermal camera 3 is capable of capturing the image of the plurality of target persons P that pass through the plurality of lanes L, respectively. As a result, the information processing system SYS may not include a plurality of thermal cameras 3 the imaging ranges TRG of which include the plurality of lanes L, respectively. Namely, the information processing system SYS may not include the plurality of thermal cameras 3 that respectively capture the images of the plurality of target persons P that respectively pass through the plurality of lanes L. Thus, a cost of the information processing system SYS is reducible.

Moreover, the information processing apparatus 4 determines one lane L through which each target person P included in the thermal image IMG T is passing. Thus, even when he thermal camera 3 captures the image of the plurality of target persons P that pass through the plurality of different lanes L, respectively, the body temperature calculation unit 413 is capable of properly calculating the body temperatures of the plurality of target persons P that pass through the plurality of different lanes L, respectively.

Moreover, the information processing apparatus 4 may divide the thermal image IMG_T into the plurality of image areas TR and determine, as one lane L through which the target person P included in the thermal image IMG T is passing, one lane L that corresponds to one image area TR of the plurality of image areas TR in which the target person P is included. In this case, the information processing apparatus 4 is capable of determining one lane L through which the target person P is passing relatively easily.

Moreover, the body temperature calculation unit 413 may calculate the timing at which the target person P included in the person image IMG P reaches the gate apparatus 1 and determine one lane L through which the target person P included in the thermal image IMG T is passing based on the calculated timing. In this case, the information processing apparatus 4 is capable of determining one lane L through which the target person P is passing relatively accurately.

Moreover, the body temperature calculation unit 413 may calculate the position of the target person P included in the thermal image IMG T in the world coordinate system based on the thermal image IMG_T and determine one lane L through which the target person P included in the thermal image IMG_T is passing based on the calculated position. In this case, the information processing apparatus 4 is capable of determining one lane L through which the target person P is passing relatively accurately.

Moreover, the body temperature calculation unit 413 may determine one lane L through which the target person P included in the thermal image IMG T is passing based on the positional relationship between the partition member BD and the target person P in the thermal image IMG T. In this case, the information processing apparatus 4 is capable of determining one lane L through which the target person P is passing relatively easily and relatively accurately.

### (4) Modified Example

Next, a modified example of the information processing system SYS will be described.

### (4-1) First Modified Example

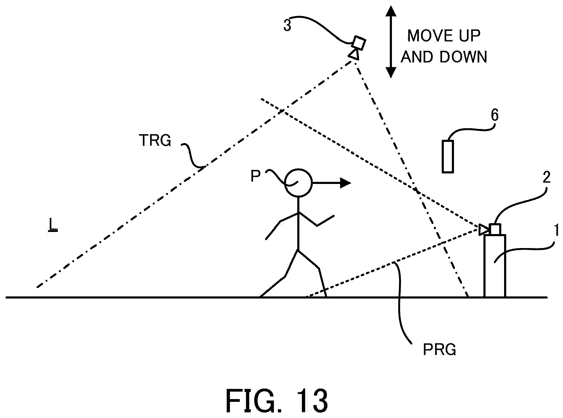

In a first modified example, as illustrated in FIG. 13 that illustrates the position of the thermal camera 3 in the first modified example, the thermal camera 3 may be placed above the plurality of lanes L. The thermal camera 3 may capture the image of the plurality of target persons P that pass through the plurality of lanes L, respectively, above the plurality of lanes L. Namely, the thermal camera 3 may capture the image of the plurality of target persons P above the plurality of target persons P. In this case, the there is a lower possibility that the thermal camera 3 is not capable of properly capturing the image of one target person P due to another target person P that is located between one target person P and the thermal camera 3, compared to a case where the thermal camera 3 captures the image of the plurality of target persons P in front of the plurality of target persons P. As a result, the thermal camera is capable of capturing the image of the plurality of target persons P that pass through the plurality of lanes L, respectively.

However, when the thermal camera 3 captures the image of the target person P above the target person P, there is a possibility that the thermal camera 3 is not capable of properly capturing the image of a surface of a body (for example, a surface of a face) of the target person P due to at least one of a hair of the target person P and a cap which the target person P wears. Thus, in the first modified example, the information processing system SYS may output a message to the target person P so that the target person P turns his face toward the thermal camera 3. For example, the information processing system SYS may include, at or near the gate apparatus 1, a display 6 that displays the message to the target person P so that the target person P turns his face toward the thermal camera 3. As a result, even when the thermal camera 3 captures the image of the target person P above the target person P, the thermal camera 3 is capable of properly capturing the image of the surface of the body of the target person P (namely, is capable of generating the thermal image IMG T that indicates the body temperature of the target person P properly).

Alternatively, the information processing system SYS may move the thermal camera 3, in addition to or instead of outputting the message to the target person P so that the target person P turns his face toward the thermal camera 3. For example, when the thermal camera 3 is not capable of properly capturing the image of the surface of the body (for example, the surface of the face) of the target person P above the target person P, the information processing system SYS may move the thermal camera 3 so that the thermal camera 3 is capable of properly capturing the image of the surface of the body (for example, the surface of the face) of the target person P in front of the target person P. Typically, the information processing system SYS may move the thermal camera 3 downward so that the thermal camera 3 is capable of properly capturing the image of the surface of the body of the target person P in front of the target person P. Then, after the thermal camera 3 captures the image of the surface of the body of the target person P in front of the target person P, the information processing system SYS may move the thermal camera 3 upward. Namely, the thermal camera 3 may be movable upward and downward. As a result, even when the thermal camera 3 captures the image of the target person P above the target person P, the thermal camera 3 is capable of properly capturing the image of the surface of the body of the target person P (namely, is capable of generating the thermal image IMG_T that indicates the body temperature of the target person P properly).

### (4-2) Second Modified Example

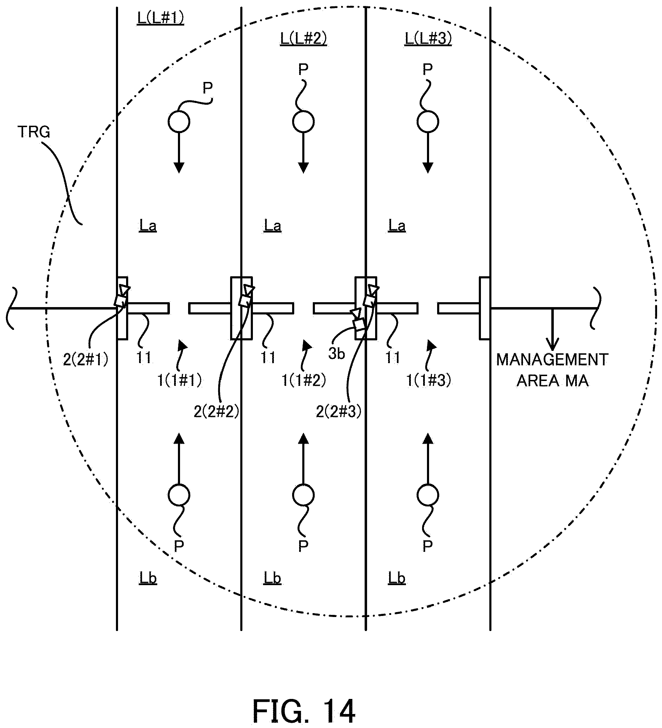

In the above described description, the target person P passing through the gate apparatus 1 moves from the lane part La to the lane part Lb of the lane L. Namely, the target person P moves toward a one-way direction. On the other hand, in a second modified example, as illustrated in FIG. 14 that conceptually illustrates a movement of the target person P in the second modified example, the target person P passing through the gate apparatus 1 may move from the lane part Lb to the lane part La of the lane L. Namely, a moving direction of the target person P may not be limited to one-way direction.

In this case, it is preferable that the thermal camera 3 capture not only the image of the target person P that passes through the lane part La but also the image of the target person P that passes through the lane part Lb. Thus, in the second modified example, as illustrated in FIG. 14, the information processing system SYS may include, as the thermal camera 3, a thermal camera 3b the imaging range TRG of which is a 360-degree range. The thermal camera 3b may include a fish-eye lens as an optical system. In this case, the thermal camera 3b is capable of capturing the image of the target person P that is passing through the lane part La before the target person P that is passing through the lane part La toward the gate apparatus 1 passes through the gate apparatus 1 and capturing the image of the target person P that is passing through the lane part Lb before the target person P that is passing through the lane part Lb toward the gate apparatus 1 passes through the gate apparatus 1.

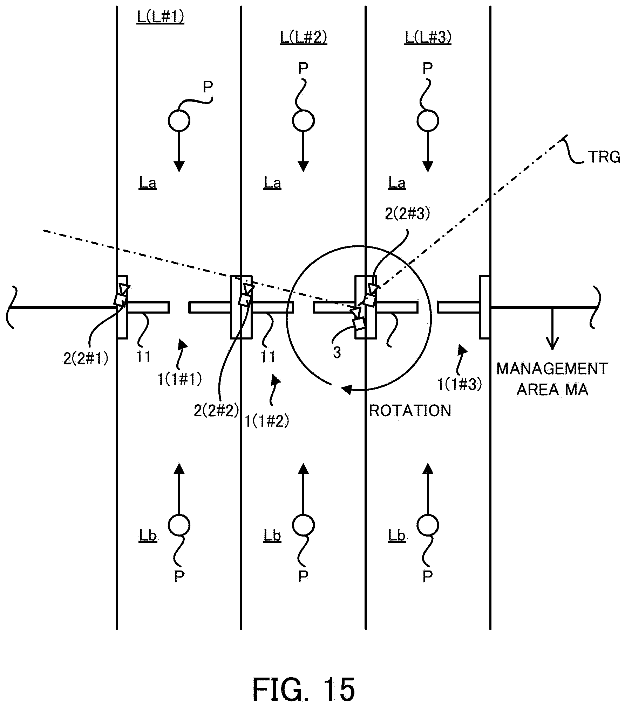

Alternatively, as illustrated in FIG. 15, the information processing system SYS may move the thermal camera 3 so that an imaging direction of the thermal camera 3 is inverted (namely, is changed). Specifically, the information processing system SYS may move the thermal camera 3 so that the imaging direction of the thermal camera 3 inverted from a direction along which the thermal camera 3 faces either one of the lane parts La and Lb to a direction along which the thermal camera 3 faces the other one of the lane parts La and Lb. As a result, the thermal camera 3 that faces the lane part La is capable of capturing the image of the target person P that is passing through the lane part La before the target person P that is passing through the lane part La toward the gate apparatus 1 passes through the gate apparatus 1. Furthermore, the thermal camera 3 that faces the lane part Lb is capable of capturing the image of the target person P that is passing through the lane part Lb before the target person P that is passing through the lane part Lb toward the gate apparatus 1 passes through the gate apparatus 1.

When the thermal camera 3 moves so that the imaging direction of the thermal camera 3 is inverted, the information processing system SYS may rotate the thermal camera 3 around a predetermined rotational axis. For example, the information processing system SYS may rotate the thermal camera 3 around a rotational axis that extends along a vertical direction.

Alternatively, even in the second modified example, the thermal camera 3 may capture the image of the target person P that passes through the lane part La but may not capture the image of the target person P that passes through the lane part Lb. In this case, the thermal camera 3 may capture the image of the target person P that is passing through the lane part La before the target person P that is passing through the lane part La toward the gate apparatus 1 passes through the gate apparatus 1 and capture the image of the target person P that is passing through the lane part La after the target person P that is passing through the lane part La to be away from the gate apparatus 1 passes through the gate apparatus 1. Namely, the thermal camera 3 may capture the image of the target person P that newly enters the management area MA and capture the image of the target person P that exits from the management area MA. In this case, the information processing apparatus 4 may perform an exit management operation of managing an exit of the target person P from the management area MA. For example, the information processing apparatus 4 may calculate, based on the thermal image IMG_T, the body temperature of the target person P that exits from the management area MA. Then, when the body temperature of the target person P that exits from the management area MA is not normal, the information processing apparatus 4 may output a message for giving notice of that fact. The information processing apparatus 4 may output a message for notifying an operator and so on of the management area MA that the body temperature of the target person P that exits from the management area MA is not normal.

Incidentally, when the thermal camera 3 captures the image of the target person P that exits from the management area MA, the thermal camera 3 captures the image of the target person P in the rear of the target person P. even in this case, the information processing apparatus 4 is capable of calculating, based on the thermal image IMG_T, the body temperature of the target person P that exits from the management area MA is not normal, as long as the surface of the body (for example, a back of a neck or a back of a hand) of the target person P is included in the thermal image IMG_T.

### (4-3) Third Modified Example

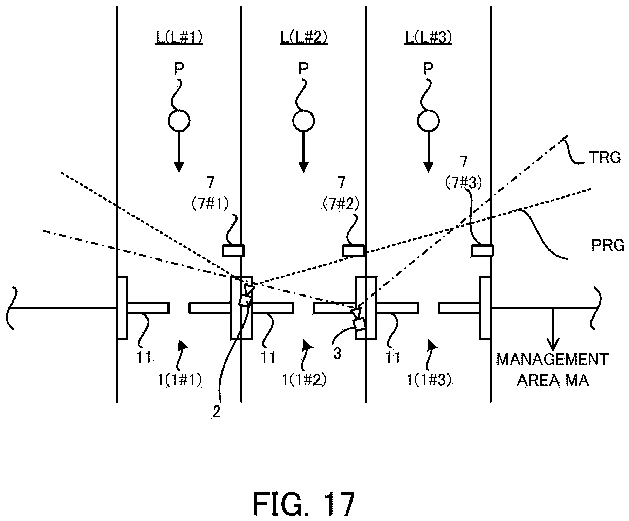

In the above described description, the information processing system SYS includes the plurality of optical cameras 2 that correspond to the plurality of gate apparatuses 1, respectively. On the other hand, in a third modified example, as illustrated in FIG. 17 that is a planar view illustrating the position of the optical camera 2 in the third modified example, the information processing system SYS may include the single that corresponds to at least two gate apparatuses 1. In an example illustrated in FIG. 17, the information processing system SYS includes the single that corresponds to the gate apparatuses 1#1 to 1#3. In this case, the optical camera 2 is placed so that at least a part of each of the lanes L#1 to L#3 is included in the imaging range RPG of the optical camera 2. Namely, the optical camera 2 is placed so that at least a part of the lane L#1, at least a part of the lane L#2 and at least a part of the lane L#3 are included in the imaging range RPG of the optical camera 2.

In this case, there is a possibility that the target person P that is passing through either one of the lanes #1 to L#3 and the target person P that is passing through another one of the lanes #1 to L#3 are included in the thermal image IMG T at the same time. Thus, in the third modified example, the information processing apparatus 4 may determine one lane L through which each target person P included in the person image IMG_P is passing through by performing an operation that is same as an operation for determining one lane L through which each target person P included in the thermal image IMG T is passing through.

Note that there is a possibility that the optical camera 2 is not capable of properly capturing the image of the surface of the body (for example, the surface of the face) of the target person P depending on a positional relationship between the optical camera 2 and the target person P. For example, in the example illustrated in FIG. 17, there is a possibility that the optical camera 2 that is placed at the lane L#2 (namely, placed at or near the gate apparatus 1#2) is not capable of properly capturing the image of the surface of the body of the target person P that passes through the lane L#1 or L#3. Thus, in the third modified example, the information processing system SYS may output a message to the target person P so that the target person P turns his face toward the optical camera 2. For example, the information processing system SYS may include, at or near the gate apparatus 1, a display 7 that displays the message to the target person P so that the target person P turns his face toward the optical camera 2. As one example, as illustrated in FIG. 17, the information processing system SYS may include, at or near the gate apparatus 1#1, a display 7#1 that displays the message to the target person P so that the target person P that passes through the lane L#1 (namely, that moves toward the gate apparatus 1#1) turns his face toward the optical camera 2. The information processing system SYS may include, at or near the gate apparatus 1#2, a display 7#2 that displays the message to the target person P so that the target person P that passes through the lane L#2 (namely, that moves toward the gate apparatus 1#2) turns his face toward the optical camera 2. The information processing system SYS may include, at or near the gate apparatus 1#3, a display 7#3 that displays the message to the target person P so that the target person P that passes through the lane L#3 (namely, that moves toward the gate apparatus 1#3) turns his face toward the optical camera 2.

However, the information processing system STYS may not all of the plurality of displays 7 that correspond to the plurality of gate apparatuses 1, respectively. For example, in FIG. 17, since the optical camera 2 is placed at or near the gate apparatus 1#2, there is a relatively high possibility that the optical camera 2 is capable of properly capturing the image of the surface of the body (for example, the surface of the face) of the target person P that passes through the lane L#2 (namely, that moves toward the gate apparatus 1#2). In this case, the information processing system SYS may not include the display 7#2.

### (4-4) Fourth Modified Example

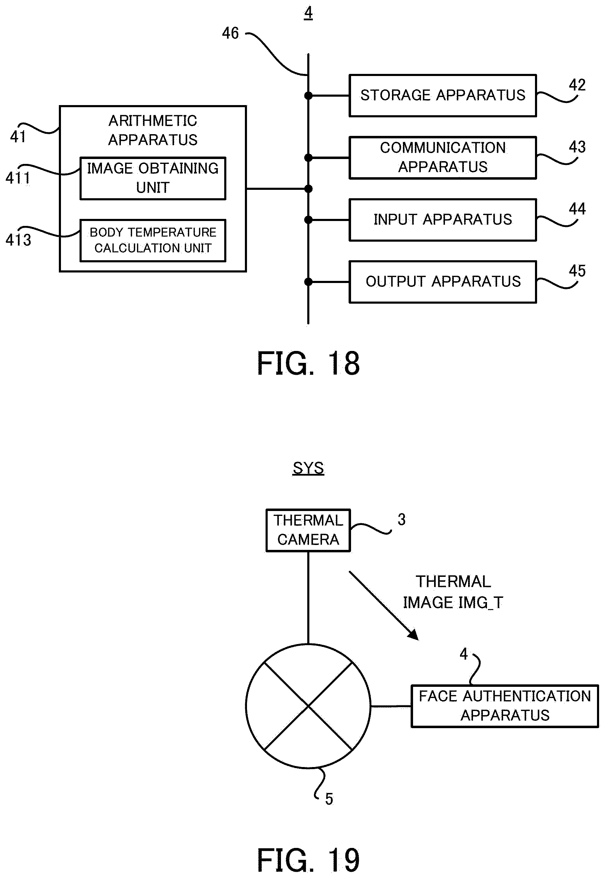

In the above described description, the information processing apparatus 4 includes the authentication unit 412 and the entry management unit 414 in order to perform the entry management operation. However, in a fourth modified example, the information processing apparatus 4 may not perform the entry management operation. In this case, as illustrated in FIG. 18, the information processing apparatus 4 may not include the authentication unit 412 and the entry management unit 414. Furthermore, the information processing apparatus 4 may not store the entry history DB 421. Furthermore, as illustrated in FIG. 19, the information processing system SYS may not include the plurality of gate apparatuses 1 and the plurality of optical cameras 2. Even in this case, the information processing apparatus 4 may obtain the thermal image IMG_T from the thermal camera 3 and determine one lane L through which the target person included in the thermal image IMG T passes.

### (5) Supplementary Note

With respect to the example embodiments described above, the following Supplementary Notes will be further disclosed.

### [Supplementary Note 1]

An information processing system including:
a plurality of gate apparatuses that are respectively placed at a plurality of lanes through each of which a target person can pass;
a thermal camera that is configured to generate a body temperature information indicating a body temperature of the target person by capturing an image of the target person included in an imaging range thereof, the imaging range including at least a part of each of the plurality of lanes; and
an information processing apparatus that obtains the body temperature information from the thermal camera.

### [Supplementary Note 2]

The information processing system according to Supplementary Note 1, wherein
the information processing apparatus includes:
an obtaining unit that obtains, as the body temperature information, a body temperature image indicating the body temperature of the target person from the thermal camera; and
determining unit that determines, as a passing lane, a lane of the plurality of lanes through which the target person indicated by the body temperature image passes.

### [Supplementary Note 3]

The information processing system according to Supplementary Note 2, wherein
the determining unit divides the body temperature image into a plurality of image areas that respectively correspond to the plurality of lanes, and determines, as the passing lane, one lane that corresponds to one image area of the plurality of image areas that includes the target person.

### [Supplementary Note 4]

The information processing system according to Supplementary Note 2 or 3, wherein
the information processing system further includes an optical camera an imaging range of which includes at least a part of a predetermined lane of the plurality of lanes and that is configured to generate a person image indicating the target person by capturing an image of the target person included in the imaging range,
the obtaining unit obtains the person image from the optical camera,
the determining unit determines a timing at which the target person indicated by the person image passes through the gate apparatus placed at the predetermined lane based on a position of a predetermined part of the target person indicated by the person image, and determines the passing lane based on the determined timing.

### [Supplementary Note 5]

The information processing system according to any one of Supplementary Notes 2 to 4, wherein
the determining unit determines a position of the target person in a world coordinate system that represents a position in a space in which the plurality of lanes are provided based on a position of a predetermined part of the target person indicated by the body temperature image, and determines the passing lane based on the determined position of the target person.

### [Supplementary Note 6]

The information processing system according to any one of Supplementary Notes 2 to 5, wherein
the plurality of lanes are separated by a partition member,
the determining unit determines the passing lane based on a position of the partition member and a position of the target person that are indicated by the body temperature image

### [Supplementary Note 7]

The information processing system according to any one of Supplementary Notes 2 to 6, wherein
the information processing system further includes an optical camera an imaging range of which includes at least a part of a predetermined lane of the plurality of lanes and that is configured to generate a person image indicating the target person by capturing an image of the target person included in the imaging range,
the information processing apparatus includes:
   an authenticating unit that authenticates the target person indicated by the person image based on the person image; and
   a generating unit that generates a management information in which an information relating to the body temperature of the target person indicated by the body temperature image and an information relating to an authenticated result of the target person when the passing lane is same as the predetermined lane.

### [Supplementary Note 8]

The information processing system according to any one of Supplementary Notes 1 to 7, wherein
the thermal camera is placed above the plurality of lanes.

### [Supplementary Note 9]

The information processing system according to Supplementary Note 8, wherein
the thermal camera is movable upward and downward.

### [Supplementary Note 10]

The information processing system according to any one of Supplementary Notes 1 to 9, wherein
the thermal camera is movable so that an imaging direction of the thermal camera is inverted.

### [Supplementary Note 11]

An information processing apparatus including:
an obtaining unit that obtains a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and
determining unit that determines, as a passing lane, a lane of the plurality of lanes through which the target person indicated by the body temperature image passes.

### [Supplementary Note 12]

An information processing method including:
obtaining a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and
determining, as a passing lane, a lane of the plurality of lanes through which the target person indicated by the body temperature image passes.

### [Supplementary Note 13]

A recording medium on which a computer program that allows a computer to execute an information processing method is recorded,
the information processing method including:
obtaining a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and
determining, as a passing lane, a lane of the plurality of lanes through which the target person indicated by the body temperature image passes.

At least a part of the feature of each embodiment described above may be combined with at least other part of the feature of each embodiment described above. A part of the feature of each embodiment described above may not be used. Moreover, the disclosures of all documents (for example, publications) that are cited in the present disclosure described above are incorporated in the present disclosure by reference if it is legally permitted.

The present disclosure is allowed to be changed, if desired, without departing from the essence or spirit of the invention which can be read from the claims and the entire specification, and an information processing system, an information processing apparatus, an information processing method and a recording medium, which involve such changes, are also intended to be within the technical scope of the present disclosure.

### Description of Reference Codes

- SYS: information processing system
- 1: gate apparatus
- 2: optical camera
- 3: thermal camera
- 4: information processing apparatus
- 41: arithmetic apparatus
- 411: image obtaining unit
- 412: authentication unit
- 413: body temperature calculation unit
- 414: entry management unit
- IMG_P: person image
- IMG_T: thermal image
- L: lane
- P: target person

## Claims

1. An information processing system comprising:
a plurality of gate apparatuses that are respectively placed at a plurality of lanes through each of which a target person can pass;
a thermal camera that is configured to generate a body temperature information indicating a body temperature of the target person by capturing an image of the target person included in an imaging range thereof, the imaging range including at least a part of each of the plurality of lanes; and
an information processing apparatus that obtains the body temperature information from the thermal camera.

2. The information processing system according to claim 1, wherein
the information processing apparatus comprises:
an obtaining unit that obtains, as the body temperature information, a body temperature image indicating the body temperature of the target person from the thermal camera; and
determining unit that determines, as a passing lane, a lane of the plurality of lanes through which the target person indicated by the body temperature image passes.

3. The information processing system according to claim 2, wherein
the determining unit divides the body temperature image into a plurality of image areas that respectively correspond to the plurality of lanes, and determines, as the passing lane, one lane that corresponds to one image area of the plurality of image areas that includes the target person.

4. The information processing system according to claim 2 or 3, wherein
the information processing system further comprises an optical camera an imaging range of which includes at least a part of a predetermined lane of the plurality of lanes and that is configured to generate a person image indicating the target person by capturing an image of the target person included in the imaging range,
the obtaining unit obtains the person image from the optical camera,
the determining unit determines a timing at which the target person indicated by the person image passes through the gate apparatus placed at the predetermined lane based on a position of a predetermined part of the target person indicated by the person image, and determines the passing lane based on the determined timing.

5. The information processing system according to any one of claims 2 to 4, wherein
the determining unit determines a position of the target person in a world coordinate system that represents a position in a space in which the plurality of lanes are provided based on a position of a predetermined part of the target person indicated by the body temperature image, and determines the passing lane based on the determined position of the target person.

6. The information processing system according to any one of claims 2 to 5, wherein
the plurality of lanes are separated by a partition member,
the determining unit determines the passing lane based on a position of the partition member and a position of the target person that are indicated by the body temperature image

7. The information processing system according to any one of claims 2 to 6, wherein
the information processing system further comprises an optical camera an imaging range of which includes at least a part of a predetermined lane of the plurality of lanes and that is configured to generate a person image indicating the target person by capturing an image of the target person included in the imaging range,
the information processing apparatus comprises:
an authenticating unit that authenticates the target person indicated by the person image based on the person image; and
a generating unit that generates a management information in which an information relating to the body temperature of the target person indicated by the body temperature image and an information relating to an authenticated result of the target person when the passing lane is same as the predetermined lane.

8. The information processing system according to any one of claims 1 to 7, wherein
the thermal camera is placed above the plurality of lanes.

9. The information processing system according to claim 8, wherein
the thermal camera is movable upward and downward.

10. The information processing system according to any one of claims 1 to 9, wherein
the thermal camera is movable so that an imaging direction of the thermal camera is inverted.

11. An information processing apparatus comprising:
an obtaining unit that obtains a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and
determining unit that determines, as a passing lane, a lane of the plurality of lanes through which the target person indicated by the body temperature image passes.

12. An information processing method comprising:
obtaining a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and
determining, as a passing lane, a lane of the plurality of lanes through which the target person indicated by the body temperature image passes.

13. A recording medium on which a computer program that allows a computer to execute an information processing method is recorded,
the information processing method comprising:
obtaining a body temperature image from a thermal camera, an imaging range of the thermal camera including at least a part of each of a plurality of lanes through each of which a target person can pass and the thermal camera being configured to generate the body temperature image indicating a body temperature of the target person by capturing an image of the target person included in the imaging range; and
determining, as a passing lane, a lane of the plurality of lanes through which the target person indicated by the body temperature image passes.
